(19) Europäisches Patentamt
European Patent Office
Office européen des brevets

(11) **EP 4 670 737 A1**

(12) **EUROPEAN PATENT APPLICATION**
published in accordance with Art. 153(4) EPC

(43) Date of publication:
**31.12.2025 Bulletin 2026/01**

(21) Application number: **24760402.8**

(22) Date of filing: **21.02.2024**

(51) International Patent Classification (IPC):
*A61K 45/00* (2006.01)   *A61K 9/20* (2006.01)
*A61K 31/194* (2006.01)   *A61K 33/10* (2006.01)
*A61P 13/02* (2006.01)   *A61P 25/02* (2006.01)
*A61P 29/00* (2006.01)

(52) Cooperative Patent Classification (CPC):
A61K 9/20; A61K 31/194; A61K 33/10;
A61K 45/00; A61P 13/02; A61P 25/02; A61P 29/00

(86) International application number:
**PCT/JP2024/006205**

(87) International publication number:
**WO 2024/177104 (29.08.2024 Gazette 2024/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **24.02.2023 JP 2023027474**

(71) Applicant: **Nippon Chemiphar Co., Ltd.
Tokyo 101-0032 (JP)**

(72) Inventors:
• **NAKAMURA Hideki
  Misato-shi, Saitama 341-0005 (JP)**
• **KUNIGAMI Toshihiro
  Misato-shi, Saitama 341-0005 (JP)**

(74) Representative: **Kraus & Lederer PartGmbB
Thomas-Wimmer-Ring 15
80539 München (DE)**

(54) **PHARMACEUTICAL FOR TREATING OR PREVENTING PERIPHERAL NEUROPATHY**

(57)    Provided is a pharmaceutical composition for treating or preventing peripheral neuropathy, the pharmaceutical composition containing an alkalinizing agent. Administration of this pharmaceutical composition enables the treatment or prevention of peripheral neuropathy induced by some form of pathological change or disease or the like, and particularly diabetes. Also provided is an alkalinizing agent or a pharmaceutical composition containing an alkalinizing agent, which exhibits suppressed expression of peripheral neuropathy. Administration of this pharmaceutical composition enables suppression of expression of peripheral neuropathy.

FIG. 1

Day21

N=7-8 mean ± S.E.M. ****: p<0.0001, *; p<0.05. Unpaired-t test.

**Description**

Technical Field

The present invention relates to a novel pharmaceutical composition containing an alkalinizing agent. More specifically, the present invention relates to a pharmaceutical composition for treating or preventing peripheral neuropathy, the composition containing an alkalinizing agent.

Priority is claimed on Japanese Patent Application No. 2023-027474, filed February 24, 2023, the content of which is incorporated herein by reference.

Background Art

Peripheral neuropathy is a pathological condition that develops due to dysfunction of peripheral nerve cells, and is caused by damage to the peripheral nerve cell perikarya or axons, or to the surrounding myelin sheaths formed by Schwann cells. Peripheral neuropathy is known to have a variety of causes, including external injuries, anticancer drug administration, and metabolic diseases such as diabetes, gives rise to sensory disorders such as pain, algesia, hypoesthesia and numbness, and causes a marked deterioration in the patient's quality of life (QOL), leading to social loss.

Pain due to peripheral neuropathy is pain caused by temporary damage to, or a functional disorder in, a portion of the peripheral neurotransmitter system. The primary causes of this nerve damage are external injury or damage of peripheral nerves, nerve plexuses or nerve surrounding soft tissue. Further, pain due to peripheral neuropathy also occurs as a result of damage to the central somatosensory pathways (for example, ascending somatosensory pathways in the spinal cord, brain stem, thalamus or cortex). Pain due to peripheral neuropathy may also be induced, for example, by any of neurodegenerative disease, bone degenerative disease, metabolic disorder, cancer, infection, inflammation, post-surgical complication, external injury, radiation therapy, and anticancer drug treatment.

For example, diabetic peripheral neuropathy is peripheral neuropathy induced by diabetes, and it is known that as diabetes progresses, peripheral neuropathy such as hyperesthesia or hypoesthesia tends to develop.

Diabetic peripheral neuropathy is classified into polyneuropathy which has symptoms such as numbness, pain, dysthermesthesia or hypoesthesia of the four limbs, autonomic neuropathy which is accompanied by sweating disorder or abnormal bowel movements or the like, and mononeuropathy in which a disorder occurs in a cranial nerve, such as facial palsy and external ophthalmoplegia. Of these, polyneuropathy and autonomic neuropathy occur as a result of polyol metabolism abnormality, and the two are often jointly termed diffuse symmetric neuropathy. Further, mononeuropathy occurs as a result of vascular occlusion.

Among the above, pain in the four limbs due to polyneuropathy, which displays the earliest onset, is classified as neuropathic pain caused by dysfunction or damage of the peripheral nervous system or central nervous system itself, and is expressed mainly through symptoms of algesia or allodynia in which tactile stimuli are misinterpreted as pain.

Furthermore, polyneuropathy is the most commonly occurring neuropathy in diabetic peripheral neuropathy, with symptoms presenting in multiple locations. Polyneuropathy occurs as a result of disorder of the sensory nerves or motor nerves, and in the case of sensory neuropathy, can result in dysesthesia (algesia, hypoesthesia, numbness (including a throbbing sensation), cold sensation, burning sensation, and formication (sensation of crawling insects) and the like), and pain (including sciatica, trigeminal neuralgia, intercostal neuralgia, or neuralgia in the four limbs). The pain frequently presents as throbbing pain accompanying numbness, and is often more intense at night. Symptoms often start with pain or numbness at the extremities of the hands and feet, in the portions typically covered with socks or gloves, with desensitization that can feel like thin sheets of paper adhered to the soles of the feet (stocking glove neuropathy). Symptoms then spread toward the center of the body, from the feet into the knees, and from the hands into the arms. Furthermore, this pain is also neuropathic pain. Motor nerve disorder tends to appear after sensory nerve disorder, and leads to a decrease in muscle strength and muscular atrophy. Specific examples include atrophy or decreased strength of the muscles of the gluteal region and the femoral region and associated pain.

In the guidelines, tricyclic antidepressants are stated as having a significant analgesic effect compared with placebo against an extremely wide range of peripheral and central neuropathic pain. However, tricyclic antidepressants have been reported as increasing the incidence of falls and sudden cardiac death in elderly patients, and should therefore be used carefully, starting with low dosages. Duloxetine is the only tricyclic antidepressant listed in the guidelines for which there is moderate evidence of efficacy (Non-Patent Document 1).

However, duloxetine has been reported as having various side effects, such as giving rise to suicidal thoughts and suicide attempts, whereas stopping administration (and particularly stopping suddenly) can lead to dizziness, headaches and nausea, and it is well known that careful administration is necessary (Non-Patent Document 2).

On the other hand, alkalinizing agents including some citric acid formulations or sodium bicarbonate are known to be effective against the representative lifestyle related disease of hyperuricemia or gout. Further, administration of an alkalinizing agent is known to suppress the production of urinary stones by making acidic urine more alkaline. Furthermore, it is known that administration of an alkalinizing agent to early-stage CKD patients suppresses the progression of kidney damage, and reduces the concentration of uremic substances in the blood (Patent Documents 1 and 2). Moreover, citric acid formulations are known to suppress renal fibrosis in diabetic nephropathy (Patent Document 3).

However, the effectiveness of alkalinizing agents in the prevention or treatment of peripheral neuropathy is unknown.

Citation List

Patent Documents

Patent Document 1
WO 2018/193648
Patent Document 2
WO 2018/193752
Patent Document 3
WO 2020/080451

Non Patent Documents

Non Patent Document 1
Guidelines for the Pharmacologic Management of Neuropathic Pain, Second Edition
Non Patent Document 2
Duloxetine Hydrochloride Capsule Cymbalta Medicine Interview Form Revised in January 2017 (Revised 11th edition)
Non Patent Document 3
Chaplan SR, Bach FW, Pogrel JW, Chung JM, Yaksh TL: Quantitative assessment of tactile allodynia in the rat paw. J Neurosci Methods. 1994 Jul; 53(1): 55 to 63.

Summary of Invention

Problems to be solved by Invention

One object of the present invention is to provide a pharmaceutical that is useful for treating or preventing peripheral neuropathy.

Another object of the present invention is to provide a pharmaceutical composition which contains an alkalinizing agent and exhibits suppressed expression of peripheral neuropathy.

Yet another object of the present invention is to provide a method for treating or preventing peripheral neuropathy induced by some form of pathological change or disease or the like.

Yet another object of the present invention is to provide a method for treating or preventing peripheral neuropathy induced by diabetes.

Yet another object of the present invention is to provide a method for treating peripheral neuropathy that exhibits suppressed expression of peripheral neuropathy. Means to Solve the Problems

As a result of intensive investigation aimed at achieving the above objects, the inventors of the present invention discovered that a specific alkalinizing agent was useful in treating or preventing peripheral neuropathy, enabling them to complete the present invention.

In other words, the present invention has the following aspects.

(1) A pharmaceutical composition for treating or preventing peripheral neuropathy, the composition containing an alkalinizing agent.
(2) The pharmaceutical composition according to (1), wherein the peripheral neuropathy is pain, sensory disturbance, mobility impairment, autonomic neuropathy, or a complication thereof attributable to peripheral neuropathy.
(3) The pharmaceutical composition according to (1) or (2), wherein the peripheral neuropathy is peripheral neuropathic pain.

(4) The pharmaceutical composition according to (3), wherein the peripheral neuropathic pain is shooting pain, burning pain, pain (excluding shooting pain and burning pain), or tingling.

(5) The pharmaceutical composition according to (1) or (2), wherein the peripheral neuropathy is a sensory disturbance attributable to peripheral neuropathy.

(6) The pharmaceutical composition according to (5), wherein the sensory disturbance attributable to peripheral neuropathy is hypoesthesia, hyperesthesia or a sensory disorder.

(7) The pharmaceutical composition according to (6), wherein the hyperesthesia is allodynia, and the sensory disorder is dysesthesia or paresthesia.

(8) The pharmaceutical composition according to (1) or (2), wherein the peripheral neuropathy is autonomic neuropathy attributable to peripheral neuropathy.

(9) The pharmaceutical composition according to (8), wherein a symptom caused by the autonomic neuropathy is urinary disturbance, sweating disorder, orthostatic hypotension, constipation, or paralytic ileus.

(10) The pharmaceutical composition according to any one of (1) to (9), wherein the peripheral neuropathy is peripheral neuropathy having symptoms that manifest in the four limbs.

(11) The pharmaceutical composition according to any one of (1) to (10), wherein the alkalinizing agent is a pharmaceutically acceptable salt of citric acid or a hydrate thereof, or a mixture thereof.

(12) The pharmaceutical composition according to any one of (1) to (11), wherein the alkalinizing agent is sodium citrate, potassium citrate, a hydrate of sodium citrate or potassium citrate, or a mixture thereof.

(13) The pharmaceutical composition according to any one of (1) to (12), wherein the alkalinizing agent contains a mixture of sodium citrate or a hydrate thereof, and potassium citrate or a hydrate thereof.

(14) The pharmaceutical composition according to any one of (1) to (13), wherein the alkalinizing agent is sodium citrate or a hydrate thereof.

(15) The pharmaceutical composition according to any one of (1) to (14), wherein the pharmaceutical composition is a tablet.

(16) The pharmaceutical composition according to any one of (1) to (10), wherein the alkalinizing agent is sodium bicarbonate.

(17) The pharmaceutical composition according to any one of (1) to (16), wherein the peripheral neuropathy is diabetic peripheral neuropathy.

Effects of Invention

The pharmaceutical composition, combination formulation and pharmaceutical kit provided by the present invention enable the treatment or prevention of peripheral neuropathy. Furthermore, the pharmaceutical composition and pharmaceutical kit provided by the present invention enable treatment or prevention to be conducted with good suppression of peripheral neuropathy, and particularly peripheral neuropathic pain.

Brief Description of the Drawings

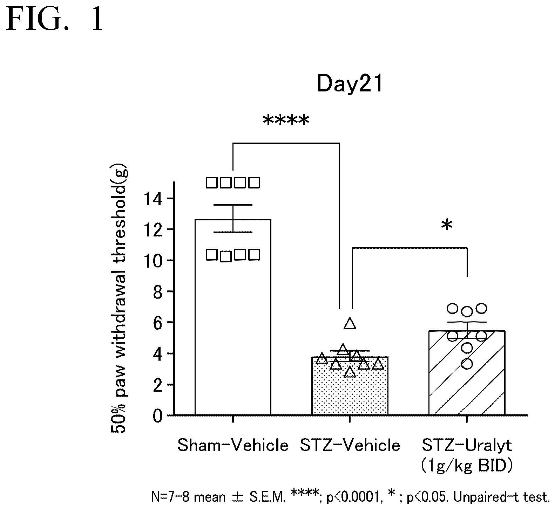

FIG. 1 is a diagram illustrating the effect of an alkalinizing agent against neuropathic pain in STZ-induced diabetes model rats, and shows the pain-related scores for each test group in relation to mechanical allodynia on day 21 from the start of STZ administration.

Description of Embodiments

1. Pharmaceutical Composition

<Pharmaceutical Composition for Treating or Preventing Peripheral Neuropathy>

A pharmaceutical composition, provided in the present invention, for treating or preventing peripheral neuropathy may contain an alkalinizing agent as an active ingredient.

The expression "contain an alkalinizing agent as an active ingredient" means that in the pharmaceutical composition, the alkalinizing agent is either a main component, or functions as a substantial active component in the treatment or prevention of peripheral neuropathy and is included in an amount that is effective for that treatment or prevention.

Alkalinizing agents are drugs that have the ability to increase the $HCO_3^-$ concentration or pH in the bodily fluids such as the blood or urine of mammals (and particularly humans). Examples of alkalinizing agents include pharmaceutically acceptable salts of citric acid or hydrates of those salts, or mixtures thereof, and sodium bicarbonate (baking soda). Examples of the pharmaceutically acceptable salts of citric acid include alkali metal citrate salts. Specific examples of these alkali metal citrate salts include potassium citrate and sodium citrate, which may exist as stable hydrates such as potassium citrate monohydrate ($C_6H_5K_3O_7 \cdot H_2O$) and sodium citrate dihydrate ($C_6H_5Na_3O_7 \cdot 2H_2O$) respectively.

Examples of preferred alkalinizing agents for inclusion in the pharmaceutical composition provided by the present invention include sodium citrate, potassium citrate, the hydrates of these salts, or mixtures thereof, and for example, a mixture of potassium citrate monohydrate ($C_6H_5K_3O_7 \cdot H_2O$) and sodium citrate dihydrate ($C_6H_5Na_3O_7 \cdot 2H_2O$) may be used. The mixing ratio between the potassium citrate monohydrate ($C_6H_5K_3O_7 \cdot H_2O$) and sodium citrate dihydrate ($C_6H_5Na_3O_7 \cdot 2H_2O$) may be determined appropriately by a person skilled in the art, and for example, the molar ratio between the potassium citrate monohydrate and the sodium citrate dihydrate may be set such that for each 1 mol of potassium citrate monohydrate, the amount of sodium citrate dihydrate is within a range from 0.01 to 100 mol. The molar ratio between the potassium citrate (for example, potassium citrate monohydrate) and the sodium citrate (for example, sodium citrate dihydrate) may be set appropriately by a person skilled in the art, and may be within a range from 0.85:1.15 to 1.15:0.85, from 0.90:1.10 to 1.10:0.90, from 0.95:1.05 to 1.05:0.95, or from 0.99:1.01 to 1.01:0.99, and is preferably 1:1.

Further, another example of the active ingredient included in the pharmaceutical composition provided by the present invention is sodium citrate or the hydrate thereof, and for example, sodium citrate dihydrate ($C_6H_5Na_3O_7 \cdot 2H_2O$) may be used.

Furthermore, yet another example of the active ingredient included in the pharmaceutical composition provided by the present invention is potassium citrate or the hydrate thereof, and for example, potassium citrate monohydrate ($C_6H_5K_3O_7 \cdot H_2O$) may be used.

In one embodiment, the alkalinizing agent included in the pharmaceutical composition of the present invention may contain a mixture of sodium citrate or a hydrate thereof, and potassium citrate or a hydrate thereof.

In one embodiment, the alkalinizing agent included in the pharmaceutical composition of the present invention may be a mixture of potassium citrate, sodium citrate, and citric acid (for example, anhydrous citric acid). In such a case, the mixing ratio (molar ratio) between the citric acid (for example, anhydrous citric acid), the potassium citrate and the sodium citrate may be determined appropriately by a person skilled in the art, and for example, may be within a range from 1:1.7 to 2.3:1.7 to 2.3, from 1:1.9 to 2.1:1.9 to 2.1, or from 1:1.95 to 2.05:1.95 to 2.05, and is preferably 1:2:2.

In one embodiment, the alkalinizing agent included in the pharmaceutical composition of the present invention may be a mixture of potassium citrate monohydrate ($C_6H_5K_3O_7 \cdot H_2O$), sodium citrate dihydrate ($C_6H_5Na_3O_7 \cdot 2H_2O$), and anhydrous citric acid. In such a case, the mixing ratio (molar ratio) between the anhydrous citric acid, the potassium citrate monohydrate ($C_6H_5K_3O_7 \cdot H_2O$) and the sodium citrate dihydrate ($C_6H_5Na_3O_7 \cdot H_2O$) may be determined appropriately by a person skilled in the art, and for example, may be within a range from 1:1.7 to 2.3:1.7 to 2.3, from 1:1.9 to 2.1:1.9 to 2.1, or from 1:1.95 to 2.05:1.95 to 2.05, and is preferably 1:2:2.

In one embodiment, the alkalinizing agent included in the pharmaceutical composition of the present invention may be composed solely of a mixture of sodium citrate or a hydrate thereof and potassium citrate or a hydrate thereof.

In this description, when weights are mentioned for citric acid, a pharmaceutically acceptable salt of citric acid or a hydrate thereof, or a mixture thereof (for example, potassium citrate monohydrate ($C_6H_5K_3O_7 \cdot H_2O$) and sodium citrate dihydrate ($C_6H_5Na_3O_7 \cdot 2H_2O$)), those weights may refer to dry weights.

In one embodiment, treatment or prevention of peripheral neuropathy may refer to the treatment or prevention of an organic disorder of the peripheral nerves.

In another embodiment, treatment or prevention of peripheral neuropathy may refer to the treatment or prevention of symptoms attributable to peripheral neuropathy, such as pain, sensory disturbance, mobility impairment, autonomic neuropathy, or a complication thereof.

In one embodiment, the above term "peripheral neuropathy" may refer to pain, sensory disturbance, mobility impairment, autonomic neuropathy, or a complication thereof attributable to peripheral neuropathy.

In another embodiment, the term "peripheral neuropathy" may refer to peripheral neuropathic pain.

Further, in yet another embodiment, the above term "peripheral neuropathic pain" may refer to shooting pain, burning pain, pain (excluding shooting pain and burning pain), or tingling.

In one embodiment, the above term "peripheral neuropathy" may refer to a sensory disturbance such as hypoesthesia, hyperesthesia or a sensory disorder that is attributable to peripheral neuropathy. Hyperesthesia is a general concept that includes allodynia, and is a concept that includes mechanical allodynia caused by mechanical stimulus. Further, the above term "sensory disorder" is a general concept that includes dysesthesia and paresthesia, and is a concept that includes cold dysesthesia caused by cold stimulus. Further, one aspect of sensory disturbance and/or mobility impairment is numbness. Accordingly, the pharmaceutical composition provided by the present invention can be used for treating or preventing numbness attributable to peripheral neuropathy.

In one embodiment, the above term "peripheral neuropathy" may refer to autonomic neuropathy attributable to peripheral neuropathy. Typical symptoms caused by autonomic neuropathy include urinary disturbance, sweating disorder, orthostatic hypotension, constipation, or paralytic ileus.

In one embodiment, the above term "peripheral neuropathy" may refer to the manifestation of symptoms in the four limbs of a mammal (and particularly a human), and in particular the manifestation of symptoms at the limb extremities.

In one embodiment, administration of the pharmaceutical composition provided by the present invention and containing an alkalinizing agent as an active ingredient can enable treatment or prevention of peripheral neuropathy.

In one embodiment, administration of the pharmaceutical composition provided by the present invention and containing an alkalinizing agent as an active ingredient can enable treatment or prevention of pain, sensory disturbance, mobility impairment, autonomic neuropathy, or a complication thereof attributable to peripheral neuropathy.

In this description, pain attributable to peripheral neuropathy is sometimes referred to as peripheral neuropathic pain.

There are no particular limitations on the types or degree of peripheral neuropathic pain that can be treated or prevented by the pharmaceutical composition provided by the present invention and containing an alkalinizing agent as an active ingredient, but typically, pain described as shooting pain, burning pain, pain (excluding shooting pain and burning pain), or tingling can be treated or prevented.

In this description, sensory disturbance attributable to peripheral neuropathy is a general concept that includes hypoesthesia, hyperesthesia and sensory disorders. Further, hyperesthesia includes allodynia, and sensory disorder is a concept that includes dysesthesia and paresthesia. Accordingly, administration of the pharmaceutical composition provided by the present invention and containing an alkalinizing agent as an active ingredient can enable treatment or prevention of sensory disturbance such as hypoesthesia, hyperesthesia and sensory disorders, including allodynia, dysesthesia and paresthesia, attributable to peripheral neuropathy.

In one embodiment, administration of the pharmaceutical composition provided by the present invention and containing an alkalinizing agent as an active ingredient can enable treatment or prevention of symptoms attributable to autonomic neuropathy, such as urinary disturbance, sweating disorder, orthostatic hypotension, constipation, or paralytic ileus.

In one embodiment, administration of the pharmaceutical composition provided by the present invention and containing an alkalinizing agent as an active ingredient can enable treatment or prevention of symptoms that manifest in the four limbs of a mammal (and particularly a human), and particularly symptoms that manifest at the limb extremities.

In this description, the term "treatment" is a concept that incorporates the elimination of, complete recovery from, curing of, or remission from a "pathological" or "abnormal" symptom, state or disease, and actions or measures therefor, incorporates the "suppression" of any worsening of a "pathological" or "abnormal" symptom, state or disease, and actions or measures therefor, and also incorporates the concept of "improvement".

In one embodiment, the term "treatment" refers to the elimination of, complete recovery from, curing of, or remission from a "pathological" or "abnormal" symptom, state or disease, or an action or measure for achieving that outcome. In another embodiment, the term "treatment" refers to the elimination of, complete recovery from, curing of, or remission from a "pathological" or "abnormal" symptom, state or disease.

In this description, the term "suppression" is a concept that incorporates halting, reducing the speed of, or decreasing the worsening or progression of a symptom, state or disease, and actions or measures therefor, or incorporates improvement in a symptom, state or disease, and actions or measures therefor. Here, the term "improvement" includes the change in a "pathological" or "abnormal" symptom, state or disease toward a more "healthy" or "normal" state, and actions or measures therefor, or achieving a "healthy" or "normal state", and actions or measures therefor. Accordingly, in one embodiment, the term "improvement" includes a change in a numerical value that acts as an indicator of a "pathological" or "abnormal" symptom or state, with the numerical value becoming smaller or larger, and either approaching a normal value or reaching a normal value, as a result of the "improvement". The above expression "worsening or progression of a symptom, state or disease" includes the worsening or progression of a "pathological" or "abnormal" symptom, state or disease, and worsening or progression from a "healthy" or "normal" state to a "pathological" or "abnormal" symptom, state or disease. In one embodiment, "the term "suppression" refers to halting, reducing the speed of, or decreasing the worsening or progression of a symptom, state or disease, or an action or measure therefor. In another embodiment, the term "suppression" refers to halting, reducing the speed of, or decreasing the worsening or progression of a symptom, state or disease.

In this description, the term "healthy" describes a state of having no acute or chronic diseases or disorders, and the term "normal" describes a state typically expressed by a healthy subject.

In this description, the term "prevention" is a concept that incorporates preventing onset of a "pathological" or "abnormal" symptom, state or disease before it occurs, and actions or measures therefor. Accordingly, in a state with no onset (also referred to as expression) of a "pathological" or "abnormal" symptom, state or disease, "suppression" of this symptom, state or disease may include the concept of "prevention".

The symptom, state or disease mentioned above is either compared before administration and then after administration of the pharmaceutical composition provided by the present invention, or compared for the case when the pharmaceutical composition provided by the present invention is administered and the case when a control or a placebo is administered.

Accordingly, for example, the expression "treatment of peripheral neuropathic pain" is a concept that includes the elimination of, complete recovery from, curing of, or remission from the peripheral neuropathic pain induced by some form of pathological change or disease or the like, and actions or measures therefor, includes suppression of the peripheral neuropathic pain induced by some form of pathological change or disease or the like, and actions or measures therefor, or includes improvement in the peripheral neuropathic pain induced by some form of pathological change or disease or the like. In another aspect, the above treatment is a concept that incorporates suppression of a symptom or state of the above peripheral neuropathic pain by administration of the pharmaceutical composition provided by the present invention and containing an alkalinizing agent as an active ingredient, compared with a placebo administration or a control.

Furthermore, for example, the expression "prevention of peripheral neuropathic pain" is a concept that incorporates preventing onset of peripheral neuropathic pain induced by some form of pathological change or disease or the like before it occurs, and actions or measures therefor, and also incorporates suppressing the pain prior to pain onset, and actions or measures therefor. In another aspect, when the pharmaceutical composition provided by the present invention and containing an alkalinizing agent as an active ingredient is administered prior to the onset of peripheral neuropathic pain induced by some form of pathological change or disease or the like, even if the pain does occur, provided a symptom or state of that pain is suppressed compared with a placebo administration or a control, that phenomenon may be included within the definition of prevention.

In the case of "pain", the effect of the above "suppression" can be evaluated based on the "pain-related score" described below compared with a placebo administration or control.

By substituting the term "pain" above with a symptom or state of peripheral neuropathy described above, the concepts of treatment and prevention can be understood. In terms of evaluation of effects, other appropriate indicators may be applied in place of the above "pain-related score".

In this description, an expression "A, B and/or C" means "at least one item selected from the group consisting of A, B and C".

The pharmaceutical composition provided by the present invention and containing an alkalinizing agent as an active ingredient is administered either orally or parenterally to a human or other mammal, wherein examples of parenteral administration include intravenous administration, subcutaneous administration, intramuscular administration, intra-articular administration, transmucosal administration, transdermal administration, transnasal administration, rectal administration, intrathecal administration, intraperitoneal administration, and local administration.

The pharmaceutical composition provided by the present invention and containing an alkalinizing agent as an active ingredient may be prepared using citric acid, a pharmaceutically acceptable salt of citric acid or a hydrate thereof, or a mixture thereof; or sodium bicarbonate, either without any other components, or by mixing with a pharmaceutically acceptable carrier such as an excipient (for example, lactose, D-mannitol, crystalline cellulose, or glucose), a binder (for example, hydroxypropyl cellulose (HPC), gelatin, or polyvinylpyrrolidone (PVP)), a lubricant (for example, magnesium stearate or talc), a disintegrant (for example, starch or carboxymethyl cellulose calcium (CMC-Ca)), or a diluent (for example, injection water or physiological saline solution), and any of various additives where necessary (for example, a pH regulator, surfactant, solubilizer, preservative, emulsifier, isotonizing agent, or stabilizer), and may have any of various forms, such as tablets, capsules, suspensions, injections or suppositories. For example, a tablet may be prepared by mixing citric acid, a pharmaceutically acceptable salt of citric acid or a hydrate thereof, or a mixture thereof, or sodium bicarbonate with an excipient (for example, lactose, D-mannitol, crystalline cellulose, or glucose), a disintegrant (for example, starch or carboxymethyl cellulose calcium (CMC-Ca)), a binder (for example, hydroxypropyl cellulose (HPC), gelatin, or polyvinylpyrrolidone (PVP)) and a lubricant (for example, magnesium stearate or talc) and the like, and then tableting the mixture.

A tablet according to the present invention is described below in further detail.

In one embodiment, the pharmaceutical composition provided by the present invention and containing an alkalinizing agent as an active ingredient is a tablet. The tablet provided by the present invention may contain an active ingredient (for example, potassium citrate or a hydrate thereof, sodium citrate or a hydrate thereof, a mixture of potassium citrate monohydrate and sodium citrate dihydrate, or sodium bicarbonate), as well as any typical pharmaceutically acceptable additives used in the field of drug production. Examples of these additives include excipients, binders, disintegrants, fluidizing agents, flavorings, lubricants, pH regulators, surfactants, stabilizers, and fragrances.

The amount of the active ingredient in the tablet provided by the present invention may typically be within a range from 10 to 95% by weight relative to the tablet, and is preferably within a range from 30 to 90% by weight, and more preferably from 60 to 85% by weight.

Examples of excipients that may be used in the tablet provided by the present invention include sugars such as lactose (for example, lactose hydrate and anhydrous lactose), glucose, sucrose, fructose and maltose, sugar alcohols such as erythritol, sorbitol, maltitol, xylitol and D-mannitol, as well as starches (for example, corn starch, potato starch, rice starch and wheat starch), crystalline cellulose, magnesium aluminometasilicate, anhydrous calcium phosphate, precipitated calcium carbonate, calcium silicate, calcium lactate and ethyl cellulose, and crystalline cellulose is particularly preferred.

The amount of the excipient in the tablet provided by the present invention may typically be within a range from 1 to 95% by weight relative to the tablet, and is preferably within a range from 1 to 80% by weight, more preferably from 3 to 80% by weight, and even more preferably from 3 to 20% by weight.

Examples of binders that may be used in the tablet provided by the present invention include hydroxypropyl cellulose, hydroxypropyl methylcellulose, polyvinylpyrrolidone, dextrin, methyl cellulose, polyvinyl alcohol, sodium alginate, aminoalkyl methacrylate copolymer, polyethylene glycol, pregelatinized starch (such as partially pregelatinized starch), agar and gelatin, and hydroxypropyl cellulose is particularly preferred.

The amount of the binder in the tablet provided by the present invention may typically be within a range from 0.1 to 30% by weight relative to the tablet, and is preferably within a range from 0.1 to 10% by weight, and more preferably from 0.3 to 3% by weight.

Examples of disintegrants that may be used in the tablet provided by the present invention include croscarmellose sodium, carmellose calcium, carboxymethyl starch sodium, low-substituted hydroxypropyl cellulose, crospovidone, starch (such as wheat starch, corn starch and partially pregelatinized starch) and carmellose, and partially pregelatinized starch is particularly preferred.

The amount of the disintegrant in the tablet provided by the present invention may typically be within a range from 0.3 to 20% by weight relative to the tablet, and is preferably within a range from 1 to 10% by weight, and more preferably from 3 to 10% by weight.

Examples of fluidizing agents that may be used in the tablet provided by the present invention include light anhydrous silicic acid, talc, and magnesium aluminometasilicate.

The amount of the fluidizing agent in the tablet provided by the present invention may typically be within a range from 0.03 to 3% by weight relative to the tablet, and is preferably within a range from 0.1 to 3% by weight, and more preferably from 0.3 to 3% by weight.

Examples of flavorings that may be used in the tablet provided by the present invention include acidifiers such as citric acid (for example, anhydrous citric acid), malic acid, acetic acid, tartaric acid, fumaric acid and ascorbic acid (however, these flavorings are not included in the active ingredient of the present invention), and sweeteners such as saccharin sodium, dipotassium glycyrrhizate, aspartame (a registered trademark), stevia, thaumatin and sucralose.

The amount of the flavoring in the tablet provided by the present invention may typically be within a range from 0.03 to 3% by weight relative to the tablet, and is preferably within a range from 0.1 to 3% by weight, and more preferably from 0.3 to 3% by weight.

Examples of lubricants that may be used in the tablet provided by the present invention include magnesium stearate, calcium stearate, talc, light anhydrous silicic acid, sucrose fatty acid esters, carnauba wax, macrogol and sodium stearyl fumarate, and magnesium stearate is particularly preferred.

The amount of the lubricant in the tablet provided by the present invention may typically be within a range from 0.1 to 30% by weight relative to the tablet, and is preferably within a range from 0.3 to 10% by weight, and more preferably from 1 to 3% by weight.

Examples of pH regulators that may be used in the tablet provided by the present invention include citric acid, phosphate salts (such as sodium dihydrogen phosphate and potassium dihydrogen phosphate), carbonate salts (such as magnesium carbonate and sodium carbonate), tartrate salts, fumarate salts, acetate salts, and amino acid salts (although the pH regulator is not included in the active ingredient of the present invention).

The amount of the pH regulator in the tablet provided by the present invention may typically be within a range from 0.1 to 30% by weight relative to the tablet, and is preferably within a range from 0.3 to 10% by weight, and more preferably from 1 to 5% by weight.

Examples of surfactants that may be used in the tablet provided by the present invention include sodium lauryl sulfate, polysorbate, sucrose fatty acid esters, polyoxyethylene hydrogenated castor oil, polyoxyl stearate, macrogol, and poloxamer.

The amount of the surfactant in the tablet provided by the present invention may typically be within a range from 0.01 to 3% by weight relative to the tablet, and is preferably within a range from 0.03 to 1% by weight, and more preferably from 0.03 to 0.5% by weight.

Examples of stabilizers that may be used in the tablet provided by the present invention include citric acid (for example, anhydrous citric acid), malic acid, acetic acid, tartaric acid, maleic acid, ascorbic acid, sodium edetate and tocopherol (although the stabilizer is not included in the active ingredient of the present invention), and anhydrous citric acid is particularly preferred.

The amount of the stabilizer in the tablet provided by the present invention may typically be within a range from 0.01 to 30% by weight relative to the tablet, and is preferably within a range from 0.1 to 30% by weight, and more preferably from 1 to 20% by weight.

Examples of fragrances that may be used in the tablet provided by the present invention include citrus-based fragrances such as lemon, orange and grapefruit, as well as peppermint, spearmint and menthol, and these fragrances may be included in appropriate amounts in the tablet (for example, within a range from 0.01 to 1% by weight relative to the tablet, and preferably within a range from 0.01 to 0.1% by weight).

The combined amount of the active ingredient and these pharmaceutically acceptable additives in the tablet provided by the present invention must not exceed 100% by weight of the tablet.

The tablet provided by the present invention contains the components described above, and may be an uncoated tablet having no coating layer, or may be a film-coated tablet having a coating layer. The amount of the coating layer may be determined appropriately by a person skilled in the art, and for example, may be within a range from 0.1 to 10% by weight relative to the uncoated tablet. The coating layer may include, in addition to the coating base, appropriate amounts of a plasticizer, colorant, and/or gloss agent or the like.

Examples of coating bases that may be used on the tablet provided by the present invention include hydroxypropyl cellulose, hydroxypropyl methylcellulose, ethyl cellulose, cellulose acetate phthalate, methacrylic acid copolymers and polyvinylpyrrolidone, and hydroxypropyl methylcellulose is particularly preferred. The amount of the coating base in the tablet provided by the present invention is typically within a range from 0.01 to 10% by weight relative to the tablet, and is preferably within a range from 0.3 to 3% by weight.

Examples of coating plasticizers that may be used in the tablet provided by the present invention include triethyl citrate, medium chain fatty acid triglycerides, triacetin, glycerol, propylene glycol and polyethylene glycol (for example, macrogol 6000), and macrogol 6000 is particularly preferred. The amount of the coating plasticizer in the tablet provided by the present invention is typically within a range from 0.01 to 1% by weight relative to the tablet, and is preferably within a range from 0.03 to 3% by weight.

Examples of coating colorants that may be used in the tablet provided by the present invention include titanium oxide, yellow iron sesquioxide, iron sesquioxide, black iron oxide, food blue No. 2, and food blue No. 2 aluminum chelate. The amount of the coating colorant in the tablet provided by the present invention is typically within a range from 0.01 to 1% by weight relative to the tablet, and is preferably within a range from 0.03 to 3% by weight.

Examples of coating gloss agents that may be used in the tablet provided by the present invention include carnauba wax. The amount of the coating gloss agent in the tablet provided by the present invention is typically within a range from 0.0001 to 0.1% by weight relative to the tablet, and is preferably within a range from 0.001 to 0.01% by weight.

The pharmaceutical composition provided by the present invention and containing an alkalinizing agent as an active ingredient can be produced using known methods from the field of drug production. For example, in the case of tablets, the production method may include a mixing step of mixing the active ingredient (for example, potassium citrate or a hydrate thereof, sodium citrate or a hydrate thereof, a mixture of potassium citrate monohydrate and sodium citrate dihydrate, or sodium bicarbonate) with the additives, a granulation step, a tableting step and/or a coating step.

The mixing step may include a step of mixing the active ingredient with additives such as an excipient, stabilizer, disintegrant and/or binder or the like. Further, prior to the tableting step, the production method may also include a step of mixing the mixture containing the active ingredient and the additives with a lubricant, flavoring and/or fragrance. Mixing may be conducted using a V-type mixer, W-type mixer, container mixer, tumbler mixer, or stirring mixer or the like.

The granulation step can be conducted using known granulation methods from the field of drug production. Examples of the granulation method include dry granulation methods, wet granulation methods and fluidized bed granulation methods.

In one embodiment, the mixture obtained in the mixing step or the granules obtained in the granulation step may be appropriately crushed and/or classified to obtain a mixture or granules having a desired particle size. Crushing can be conducted using the types of crushing devices known in the field of drug production, such as a ball mill, jet mill, or hammer mill or the like. Classification may be conducted using a sieve within a range from a 16-mesh sieve (mesh opening: 1,000 $\mu$m) to a 32-mesh sieve (mesh opening: 500 $\mu$m).

The tableting step can be conducted using tableting methods known in the field of drug production. Examples of the tableting method include direct tableting methods, dry tableting methods, wet tableting methods, and external lubricant tableting methods. For example, by using a tableting machine known in the field of drug production, such as a single-shot tableting machine or a rotary tableting machine, the mixture or granules obtained in the step described above can be tableted. In those cases where a single-shot tableting machine or rotary tableting machine or the like is used, a tableting pressure within a range from 1 kN to 30 kN may be used.

The coating step may be conducted using known methods from the field of drug production. For example, the coating step may be conducted by spray coating a coating liquid containing the coating base and appropriate amounts of a plasticizer, colorant and/or gloss agent and the like onto the exterior of the uncoated tablets.

In one embodiment, the tablet provided by the present invention can be produced by mixing the active ingredient, an excipient (for example, lactose, D-mannitol, crystalline cellulose and/or glucose), a binder (for example, hydroxypropyl cellulose (HPC), gelatin and/or polyvinylpyrrolidone (PVP)), a stabilizer (for example, anhydrous citric acid), a disintegrant (for example, starch (such as partially pregelatinized starch) and/or carboxymethyl cellulose calcium (CMC-Ca)), and a lubricant (for example, magnesium stearate), tableting the mixture to obtain uncoated tablets, and forming a coating layer containing a coating base (for example, hydroxypropyl cellulose, hydroxypropyl methylcellulose and/or PVP), a plasticizer (for example, triethyl citrate and/or macrogol 6000), a colorant (for example, iron sesquioxide and/or titanium oxide) and a gloss agent (for example, carnauba wax) on the exterior surfaces of the uncoated tablets.

In one embodiment, the hardness of the obtained tablets may be within a range from 10 to 200 N, and preferably from 30 to 150 N.

In the pharmaceutical composition provided by the present invention and containing an alkalinizing agent as an active ingredient, the amount of the alkalinizing agent may be set as appropriate.

In one embodiment, the amount of the alkalinizing agent in the pharmaceutical composition provided by the present invention may be set to an amount that results in an improvement in aciduria in gout or hyperuricemia when the dosage of the alkalinizing agent is administered to a human, or may be set to a smaller amount or a larger amount. For example, the amount of the alkalinizing agent may be set to a value that is within a range from 100 to 800% by mass, from 200 to 800% by mass, or from 400 to 800% by mass, of the daily dosage recognized in Japan as providing improvement in aciduria in gout or hyperuricemia (for example, in the case where a citric acid formulation is used as the alkalinizing agent: tablets each containing 231.5 mg of potassium citrate ($C_6H_5K_3O_7 \cdot H_2O$) and 195.0 mg of sodium citrate hydrate ($C_6H_5Na_3O_7 \cdot 2H_2O$) are administered orally at a rate of two tablets per dosage and three doses per day; or in the case where sodium bicarbonate is used as the alkalinizing agent: 3 to 5 g administered orally per day).

In one embodiment, the pharmaceutical composition provided by the present invention and containing an alkalinizing agent as an active ingredient may be in the form of a tablet, wherein each tablet contains potassium citrate monohydrate or sodium citrate dihydrate as the active ingredient in an amount within a range from 10 mg to 1 g, preferably from 100 mg to 500 mg, and more preferably from 400 to 500 mg.

In one embodiment, the pharmaceutical composition provided by the present invention and containing an alkalinizing agent as an active ingredient may be in the form of a tablet, wherein each tablet contains potassium citrate monohydrate and sodium citrate dihydrate in respective amounts within a range from 10 mg to 300 g and a combined amount of 20 mg to 600 mg, preferably in respective amounts from 150 mg to 250 mg and a combined amount of 400 mg to 500 mg, and more preferably in respective amounts from 190 to 240 mg and a combined amount of 400 mg to 450 mg.

In one embodiment, the pharmaceutical composition provided by the present invention and containing an alkalinizing agent as an active ingredient may be in the form of a tablet, wherein each tablet contains sodium bicarbonate as the alkalinizing agent in an amount within a range from 10 mg to 1 g, and preferably from 100 mg to 500 mg.

In one embodiment, the pharmaceutical composition provided by the present invention and containing an alkalinizing agent as an active ingredient may be in the form of a tablet, wherein each tablet contains 231.5 mg of potassium citrate monohydrate and 195.0 mg of sodium citrate dihydrate as the active ingredients, and contains anhydrous citric acid, crystalline cellulose, partially pregelatinized starch, hydroxypropyl cellulose, magnesium stearate, hypromellose, macrogol 6000, titanium oxide and carnauba wax as additives.

In one embodiment, a tablet containing 231.5 mg of potassium citrate monohydrate and 195.0 mg of sodium citrate dihydrate may be used as a single dosage unit.

In this description, a "dosage unit" indicates a unit of the formulation, wherein a "single dosage unit" represents the smallest unit of the formulation. Accordingly, in the case of tablets, the dosage unit is each tablet, and a single dosage unit indicates one tablet. In the case of an injection, the dosage unit is an injection used to fill a sealed container such as an ampule or vial, and a single dosage unit indicates the injection contained within a single sealed container such as an ampule or vial. In the case of a powder or jelly-like formulation, the dosage unit is the powder or jelly-like formulation sealed in a bag produced using a single-layer or multilayer film-like sheet of polyethylene or the like, or a similar sheet provided with an aluminum layer, and a single dosage unit indicates the powder or jelly-like formulation sealed in a single bag. In the case of capsules (hard capsules, soft capsules, and seamless capsules and the like), the dosage unit is each capsule, and a single dosage unit indicates one capsule.

In those cases where the pharmaceutical composition provided by the present invention is administered to a human or other mammal, one or two or more of the above dosage units may be administered per dose, or a single dosage unit may be divided and only a portion administered.

The dosage of the active ingredient may be set appropriately in accordance with factors such as the type of active ingredient, the administration method, the age, weight, gender, symptoms and drug sensitivity of the administration subject, the degree of pathological change or disease that may cause peripheral neuropathy, and the degree of peripheral neuropathy, and typically the dosage may be adjusted in accordance with the degree of pathological change or disease that may cause peripheral neuropathy, the degree of peripheral neuropathy, and the level of improvement in the symptoms.

In one embodiment, in the case where a mixture of potassium citrate monohydrate and sodium citrate dihydrate is administered orally as the active ingredient to a human, the potassium citrate monohydrate and sodium citrate dihydrate may be administered using respective dosages of 1.5 to 6 g/day and a combined amount of 3 to 12 g/day, respective dosages of 1.5 to 9 g/day and a combined amount of 3 to 18 g/day, or respective dosages of 1.5 to 12 g/day and a combined amount of 3 to 24 g/day, and preferably respective dosages of 3 to 12 g/day and a combined amount of 6 to 24 g/day, respective dosages of 3 to 9 g/day and a combined amount of 6 to 18 g/day, or respective dosages of 6 to 9 g/day and a combined amount of 12 to 18 g/day, wherein the administration may be performed between one and five times per day, and is preferably divided into three doses per day.

In one embodiment, in the case where either potassium citrate monohydrate or sodium citrate dihydrate is administered orally as the active ingredient to a human, administration may be conducted using a dosage of 3 to 24 g/day, 3 to 18 g/day, or 3 to 12 g/day, and the administration may be performed between one and five times per day, and is preferably divided into three doses per day.

In one embodiment, in the case where sodium bicarbonate is administered orally as the active ingredient to a human, administration may be conducted using a dosage of 2.25 to 9 g/day, and the administration may be performed between one and five times per day, and is preferably divided into three doses per day.

In one embodiment, the pharmaceutical composition provided by the present invention and containing an alkalinizing agent as an active ingredient may be administered prior to the onset of peripheral neuropathy to a subject exhibiting expression or onset of a pathological change or disease that can cause peripheral neuropathy, and therefore at risk of developing peripheral neuropathy, with the object of preventing the onset of peripheral neuropathy, may be administered prior to the onset of peripheral neuropathy and then continued after peripheral neuropathy develops, or may be administered to a subject suffering from peripheral neuropathy induced by some form of pathological change or disease or the like with the object of treating the peripheral neuropathy. The administration may be continued in accordance with the degree of pathological change or disease that can cause peripheral neuropathy, the degree of peripheral neuropathy, and the level of improvement in the symptoms.

In one embodiment, the pharmaceutical composition provided by the present invention and containing an alkalinizing agent as an active ingredient may be administered for a long period of time, and for example, may be administered for one week, 2 weeks, 3 weeks, 6 weeks, 8 weeks, 10 weeks, 12 weeks, 24 weeks, 40 weeks, 60 weeks, 80 weeks, 100 weeks, 120 weeks, at least one week, at least 2 weeks, at least 3 weeks, at least 6 weeks, at least 8 weeks, at least 10 weeks, at least 12 weeks, at least 24 weeks, at least 40 weeks, at least 60 weeks, at least 80 weeks, at least 100 weeks, at least 120 weeks, at least 6 weeks but not longer than 24 weeks, at least 12 weeks but not longer than 24 weeks, at least 6 weeks but not longer than 30 weeks, at least 12 weeks but not longer than 30 weeks, at least 6 weeks but not longer than 40 weeks, at least 12 weeks but not longer than 40 weeks, at least 6 weeks but not longer than 60 weeks, at least 12 weeks but not longer than 60 weeks, at least 6 weeks but not longer than 80 weeks, at least 12 weeks but not longer than 80 weeks, at least 6 weeks but not longer than 100 weeks, at least 12 weeks but not longer than 100 weeks, at least 6 weeks but not longer than 120 weeks, at least 12 weeks but not longer than 120 weeks, or at least 24 weeks but not longer than 120 weeks.

In one embodiment, the pharmaceutical composition provided by the present invention and containing an alkalinizing agent as an active ingredient may be evaluated for the treatment or preventive effect on peripheral neuropathy induced by some form of pathological change or disease or the like, by conducting daily continuous administration of the pharmaceutical composition from the time of expression or onset of the pathological change or disease that may cause peripheral neuropathy.

\<Pharmaceutical Composition for Treating Diabetes with Suppressed Expression of Peripheral Neuropathy Induced by Diabetes\>

One embodiment is able to provide a pharmaceutical composition for treating diabetes that exhibits suppressed expression of peripheral neuropathy induced by diabetes, wherein this pharmaceutical composition contains a diabetes treatment and an alkalinizing agent as active ingredients.

In one aspect, the expression "pharmaceutical composition for treating diabetes that exhibits suppressed expression of peripheral neuropathy induced by diabetes" describes a pharmaceutical composition for treating diabetes that exhibits suppressed expression of peripheral neuropathy compared with a placebo.

In one aspect, the expression "pharmaceutical composition for treating diabetes that exhibits suppressed expression of peripheral neuropathy induced by diabetes" describes a pharmaceutical composition for treating diabetes which, compared with a "pharmaceutical composition for treating diabetes containing no alkalinizing agent" that contains the same amount of the diabetes treatment drug as the amount of the diabetes treatment drug included in the "pharmaceutical composition for treating diabetes that exhibits suppressed expression of peripheral neuropathy induced by diabetes" provided by the present invention, exhibits suppressed expression of peripheral neuropathy induced by diabetes.

In another aspect, the "pharmaceutical composition for treating diabetes that exhibits suppressed expression of peripheral neuropathy induced by diabetes" is a pharmaceutical composition for treating diabetes for which no expression of peripheral neuropathy induced by diabetes develops.

This rate of suppression of the expression of peripheral neuropathy can be calculated, for example in the case of "pain", based on a suitable evaluation indicator such as the "pain-related score" described below.

In one embodiment, examples of the diabetes treatment drug included as an active ingredient in the "pharmaceutical composition for treating diabetes that exhibits suppressed expression of peripheral neuropathy induced by diabetes" provided by the present invention include at least one diabetes treatment drug selected from the group consisting of sulfonylurea drugs such as glibenclamide, gliclazide, and glimepiride; fastacting insulin secretagogues such as nateglinide, mitiglinide calcium hydrate, and repaglinide; DPP-4 inhibitors such as sitagliptin phosphate hydrate, vildagliptin, alogliptin benzoate, linagliptin, teneligliptin hydrobromide hydrate, anagliptin, saxagliptin hydrate, trelagliptin succinate, and omarigliptin; GLP-1 receptor agonists such as semaglutide; glimin-based drugs such as imeglimin hydrochloride; biguanide drugs such as buformin hydrochloride and metformin hydrochloride; thiazolidine drugs such as pioglitazone hydrochloride; glimin drugs such as imeglimin hydrochloride; $\alpha$-glucosidase inhibitors such as acarbose, voglibose, and miglitol; and SGLT2 inhibitors such as ipragliflozin L-proline, dapagliflozin propylene glycol hydrate, luseogliflozin hydrate, tofogliflozin hydrate, canagliflozin hydrate, and empagliflozin.

In one embodiment, examples of substances that may be used as the alkalinizing agent included as an active ingredient in the pharmaceutical composition provided by the present invention include the pharmaceutically acceptable salts of citric acid described above, hydrates of those salts, mixtures thereof, and sodium bicarbonate.

In one embodiment, by administering the pharmaceutical composition provided by the present invention to a subject such as a human or other mammal suffering from some form of pathological change or disease or the like that may cause peripheral neuropathy, expression of peripheral neuropathy can be suppressed.

In one embodiment, the pharmaceutical composition provided by the present invention is administered orally or parenterally to a subject such as a human or other mammal suffering from some form of pathological change or disease or the like that may cause peripheral neuropathy, wherein examples of the parenteral administration include intravenous administration, subcutaneous administration, intramuscular administration, intra-articular administration, transmucosal administration, transdermal administration, transnasal administration, rectal administration, intrathecal administration, intraperitoneal administration, and local administration.

In one embodiment, the formulation techniques described above in relation to the pharmaceutical composition containing an alkalinizing agent as an active ingredient can also be applied to the pharmaceutical composition provided by the present invention.

Examples of other embodiments of the present invention are described below.

<1-1> An alkalinizing agent or a pharmaceutical composition containing an alkalinizing agent, for use in treating or preventing peripheral neuropathy.

<1-2> The alkalinizing agent or pharmaceutical composition containing an alkalinizing agent for the use disclosed in <1-1>, wherein the peripheral neuropathy is pain, sensory disturbance, mobility impairment, autonomic neuropathy, or a complication thereof that is attributable to peripheral neuropathy.

<1-3> The alkalinizing agent or pharmaceutical composition containing an alkalinizing agent for the use disclosed in <1-1> or <1-2>, wherein the peripheral neuropathy is peripheral neuropathic pain.

<1-4> The alkalinizing agent or pharmaceutical composition containing an alkalinizing agent for the use disclosed in <1-3>, wherein the peripheral neuropathic pain is shooting pain, burning pain, pain (excluding shooting pain and burning pain), or tingling.

<1-5> The alkalinizing agent or pharmaceutical composition containing an alkalinizing agent for the use disclosed in <1-1> or <1-2>, wherein the peripheral neuropathy is a sensory disturbance attributable to peripheral neuropathy.

<1-6> The alkalinizing agent or pharmaceutical composition containing an alkalinizing agent for the use disclosed in <1-5>, wherein the sensory disturbance attributable to peripheral neuropathy is hypoesthesia, hyperesthesia or a sensory disorder.

<1-7> The alkalinizing agent or pharmaceutical composition containing an alkalinizing agent for the use disclosed in <1-6>, wherein the hyperesthesia is allodynia, and the sensory disorder is dysesthesia or paresthesia.

<1-8> The alkalinizing agent or pharmaceutical composition containing an alkalinizing agent for the use disclosed in <1-1> or <1-2>, wherein the peripheral neuropathy is autonomic neuropathy attributable to peripheral neuropathy.

<1-9> The alkalinizing agent or pharmaceutical composition containing an alkalinizing agent for the use disclosed in <1-8>, wherein a symptom caused by the autonomic neuropathy is urinary disturbance, sweating disorder, orthostatic hypotension, constipation, or paralytic ileus.

<1-10> The alkalinizing agent or pharmaceutical composition containing an alkalinizing agent for the use disclosed in any one of <1-1> to <1-9>, wherein the peripheral neuropathy is peripheral neuropathy having symptoms that manifest in the four limbs.

<1-11> The alkalinizing agent or pharmaceutical composition containing an alkalinizing agent for the use disclosed in any one of <1-1> to <1-10>, wherein the alkalinizing agent is a pharmaceutically acceptable salt of citric acid or a hydrate thereof, or a mixture thereof.

<1-12> The alkalinizing agent or pharmaceutical composition containing an alkalinizing agent for the use disclosed in any one of <1-1> to <1-11>, wherein the alkalinizing agent is sodium citrate, potassium citrate, a hydrate of sodium citrate or potassium citrate, or a mixture thereof.

<1-13> The alkalinizing agent or pharmaceutical composition containing an alkalinizing agent for the use disclosed in any one of <1-1> to <1-12>, wherein the alkalinizing agent contains a mixture of sodium citrate or a hydrate thereof, and potassium citrate or a hydrate thereof.

<1-14> The alkalinizing agent or pharmaceutical composition containing an alkalinizing agent for the use disclosed in any one of <1-1> to <1-13>, wherein the alkalinizing agent is sodium citrate or a hydrate thereof.

<1-15> The alkalinizing agent or pharmaceutical composition containing an alkalinizing agent for the use disclosed in any one of <1-1> to <1-14>, wherein the pharmaceutical composition is a tablet.

<1-16> The alkalinizing agent or pharmaceutical composition containing an alkalinizing agent for the use disclosed in any one of <1-1> to <1-10>, wherein the alkalinizing agent is sodium bicarbonate.

<1-17> The alkalinizing agent or pharmaceutical composition containing an alkalinizing agent for the use disclosed in any one of <1-1> to <1-16>, wherein the peripheral neuropathy is diabetic peripheral neuropathy.

<2-1> A method for treating or preventing peripheral neuropathy, the method including administering an effective amount of an alkalinizing agent to a subject requiring treatment or prevention of peripheral neuropathy.

<2-2> A method for treating peripheral neuropathy that exhibits suppressed expression of peripheral neuropathy, the method including administering an effective amount of an alkalinizing agent to a subject requiring treatment of peripheral neuropathy.

<2-3> The method disclosed in <2-1> or <2-2>, wherein the peripheral neuropathy is diabetic peripheral neuropathy.

<3-1> Use of an alkalinizing agent for producing a pharmaceutical composition for treating or preventing peripheral neuropathy.

<3-2> Use of an alkalinizing agent for producing a pharmaceutical composition that exhibits suppressed expression of peripheral neuropathy.

<3-3> The use disclosed in <3-1> or <3-2>, wherein the peripheral neuropathy is diabetic peripheral neuropathy.

4. Food Composition

In one embodiment, the present invention can provide an alkalinizing agent or a food composition containing an alkalinizing agent, for use in a non-therapeutic application that suppresses peripheral neuropathy.

The alkalinizing agents described above in the section entitled "1. Pharmaceutical Composition" may be used as the alkalinizing agent. Examples of the alkalinizing agent include pharmaceutically acceptable salts of citric acid (for example, alkali metal citrate salts or hydrates thereof, or a mixture thereof) or sodium bicarbonate, which may be used as food acceptable salts of citric acid, and a mixture of potassium citrate monohydrate ($C_6H_5K_3O_7 \cdot H_2O$) and sodium citrate dihydrate ($C_6H_5Na_3O_7 \cdot 2H_2O$), or sodium citrate dihydrate is preferred.

The amount of the citric acid, pharmaceutically acceptable salt of citric acid or hydrate thereof, mixture thereof, or sodium bicarbonate in the food composition provided by the present invention may be determined appropriately depending on the type of food. Examples of the food composition include specific health foods, foods with functional claims, food for hospital patients, and supplements. There are no particular limitations on the form of these food compositions, provided the form contains an amount of the alkalinizing agent that is effective in achieving the above effects and can be ingested orally, and typical food and beverage forms are acceptable. Among the various formulations applicable to pharmaceutical compositions, formulations that are suited to oral administration such as tablets, capsules, and suspensions and the like may be used. The composition and production method for these formulations may employ the compositions and production methods described above for pharmaceutical formulations in the section entitled "1. Pharmaceutical Composition", or may apply known formulation techniques from the field of pharmaceutical formulation techniques.

For example, in the case of a specific health food, a food with functional claims, a food for hospital patients or a supplement, the food may contain 1/3 of a combined total of 1 to 3 g of potassium citrate monohydrate and sodium citrate dihydrate as active ingredients per serving of the food. For example, when the specific health food, food with functional claims, food for hospital patients or supplement is provided in the form of tablets, a single 300 mg to 600 mg tablet may contain 70 to 80% by weight of citric acid, a pharmaceutically acceptable salt of citric acid or a hydrate thereof, or a mixture thereof.

In those cases where the food composition provided by the present invention is not provided as a formulation, but is rather provided in the form of a typical food or beverage, then depending on the type of food or beverage, a person skilled in the art can produce the food or beverage in an appropriate manner, for example, by blending citric acid, a pharmaceutically acceptable salt of citric acid or a hydrate thereof, or a mixture thereof (for example, potassium citrate and/or sodium citrate), or sodium bicarbonate into the food raw material.

Examples of the form of the food or beverage include liquid, milky or paste-like foods such as beverages, soy sauce, milk, yoghurt and miso; semi-solid foods such as jellies or soft candies; solid foods such as candy, chewing gum, tofu and supplements; and powdered foods.

Examples of the beverages include fruit juices and fruit juice beverages, coffee beverages, oolong tea beverages, green tea beverages, tea beverages, barley tea beverages, vegetable beverages, carbonated beverages such as soft drinks, fruit juice extract-containing beverages, vegetable extract-containing beverages, flavored waters, sports beverages, and diet beverages.

The beverage may also contain one additive or a mixture of additives selected from among antioxidants, fragrances, various esters, organic acids, salts of organic acids, inorganic acids, salts of inorganic acids, inorganic salts, colorants, emulsifiers, preservatives, flavorings, sweeteners, acidifiers, fruit juice extracts, vegetable extracts, nectar extracts, pH regulators, and quality stabilizers.

The food composition provided by the present invention may be used in the same manner as the methods for using the pharmaceutical composition described above in the section entitled "1. Pharmaceutical Composition", or may also be used in applications not targeting the treatment or prevention of disease (also referred to as "non-therapeutic applications"). In other words, based on the alkalinizing agent contained in the food composition provided by the present invention, by using an amount of the alkalinizing agent in the food composition that is similar to the amount of the alkalinizing agent included in the above pharmaceutical composition, the food composition can be used for targeted subjects of the pharmaceutical composition. Further, in one embodiment, the "food composition" according to the present invention may be ingested as part of a non-therapeutic application for a subject undergoing administration of a treatment or preventive drug for a disease or the like such as diabetes that can cause peripheral neuropathy, or to suppress the expression of peripheral neuropathy in a subject (such as a human or other mammal) scheduled to undergo administration of a treatment or preventive drug for a disease or the like such as diabetes that can cause peripheral neuropathy, or to suppress peripheral neuropathy that has already manifested. In such cases, the therapeutic effect of the alkalinizing agent itself is basically the same regardless of whether the alkalinizing agent is a component of a pharmaceutical composition or a component of a food composition, and therefore the amount used of the food composition and the method used for administering the food composition may be adjusted appropriately on the basis of the alkalinizing agent in order to achieve the desired effect.

Food compositions which are used with subjects (such as humans or other mammals) that do not have a "pathological" or "abnormal" symptom, state or disease, namely, subjects (such as humans or other mammals) in a "healthy" or "normal" state, for the purposes of maintaining or extending that "healthy" or "normal" state, are sometimes referred to as "foods with functional claims".

The term "administration" described above in the section entitled "1. Pharmaceutical Composition" may also be applied to the "food composition" according to the present invention, but in the case of the "food composition" according to the present invention, the term "administration" may also be replaced with the term "ingestion". Accordingly, the terms "administer" or "administered" may be replaced with an expression such as "ingest", "ingested" or "be ingested" in accordance with the context.

The present invention is described below in further detail using a series of examples, but the present invention is in no way limited by these examples.

Examples

Example 1: Evaluation of Alkalinizing Agent in STZ-Induced Diabetes Model

(Test Substance)

For the alkalinizing agent, a mixture containing, per 1 g of dry weight, 463 mg of potassium citrate monohydrate ($C_6H_5K_3O_7 \cdot H_2O$), 390 mg of sodium citrate dihydrate ($C_6H_5Na_3O_7 \cdot 2H_2O$) and 147 mg of pharmaceutically acceptable additives (Uralyt U Combination Powder, manufactured by Nippon Chemiphar Co., Ltd.) was used. Following weighing of the required amount of the Uralyt U combination powder (hereinafter also abbreviated as simply "Uralyt"), sufficient injection water was added to dissolve the powder and prepare a solution of the target concentration at the time of use.

In the Sham group and the control group, the injection water used as the solvent for the alkalinizing agent was used.

(Animals)

Eight-week old male Crl:CD (SD) rats (from The Jackson Laboratory Japan, Inc.) were used. The animals were provided with an acclimatization period of at least 6 days, a single pain evaluation was conducted during this period, and only healthy animals for which the 50% withdrawal threshold in the pain evaluation was at least 8 g were selected for testing.

(Preparation of Test Animals)

Using the method described below, test animals: streptozocin (hereinafter also abbreviated as STZ) induced diabetic peripheral neuropathy model rats were prepared.

With the day on which the STZ was administered being deemed day 1 of the test, the STZ-administered rats were grouped on day 14 of the test. Specifically, on the day of grouping, animals for which the 50% withdrawal threshold of the pain evaluation was at least 8 g and the blood glucose level was 300 mg/dL or less were excluded from the subjects for grouping, and the remaining animals were grouped using a computer program (IBUKI, from Nihon Bioresearch Inc.) to achieve substantially the same average values for the 50% withdrawal threshold, blood glucose level and body weight in each group, with 8 animals in each of the control group and the alkalinizing agent group. In total, there groups were prepared, composed of group 1: a Sham group, group 2: a control group, and group 3: an alkalinizing agent group.

The rats of group 2: the control group, and group 3: the alkalinizing agent group were administered with STZ intravenously through the tail vein. Group 1: the Sham group was administered with a 0.75 mmol/L CAB solution, also intravenously through the tail vein. In the period from day 15 to day 21 of the test, the three groups were subjected to repeated oral administration (forced oral administration) twice per day, using injection water in the case of group 1: the Sham group and group 2: the control group, and a dosage of 1 g/kg of Uralyt in the case of group 3: the alkalinizing agent group.

(Measurement Timings)

Measurements were conducted on day 21 of the test, three hours after the administration of the test substance and the administration medium. The pain evaluations were conducted blind.

(Pain Evaluations)

Each pain evaluation was conducted using the von Frey test in accordance with the method described in Non-Patent Document 3. The 50% withdrawal threshold value was calculated using the up-down stimulation method, using a von Frey filament (target force: 0.4, 0.6, 1, 2, 4, 6, 8, 15 g, manufactured by DanMic Global, LLC).

<<Method for Calculating 50% Withdrawal Threshold>>

$$50\% \text{ withdrawal threshold} = (10(Xf+k\times\delta))/10{,}000$$

Xf: Evaluator size of last used von Frey filament
k: Withdrawal reaction pattern
δ: Average difference between filaments used

Each measurement was conducted by transferring the test animal to a mesh bottom individual cage (W110 × D180 × H150 mm) used for measuring the pain-related score, and then waiting at least 30 minutes for the individual to acclimatize to the new environment before conducting the evaluation.

(Results)

Repeated administration of the Uralyt U combination powder to the STZ-induced diabetic peripheral neuropathy model rats suppressed the onset of mechanical allodynia (FIG. 1).

Based on the above results, it is thought that repeated oral administration of Uralyt U combination powder is extremely effective against STZ-induced peripheral neuropathy.

Industrial Applicability

The pharmaceutical composition provided by the present invention can treat or prevent peripheral neuropathy.

Further, the pharmaceutical composition provided by the present invention can suppress peripheral neuropathy induced by some form of pathological change or disease or the like, and particularly diabetes.

Claims

1. A pharmaceutical composition for treating or preventing peripheral neuropathy, the composition comprising an alkalinizing agent.

2. The pharmaceutical composition according to Claim 1, wherein the peripheral neuropathy is pain, sensory disturbance, mobility impairment, autonomic neuropathy, or a complication thereof attributable to peripheral neuropathy.

3. The pharmaceutical composition according to Claim 1 or 2, wherein the peripheral neuropathy is peripheral neuropathic pain.

4. The pharmaceutical composition according to Claim 3, wherein the peripheral neuropathic pain is shooting pain, burning pain, pain (excluding shooting pain and burning pain), or tingling.

5. The pharmaceutical composition according to Claim 1 or 2, wherein the peripheral neuropathy is a sensory disturbance attributable to peripheral neuropathy.

6. The pharmaceutical composition according to Claim 5, wherein the sensory disturbance attributable to peripheral neuropathy is hypoesthesia, hyperesthesia or a sensory disorder.

7. The pharmaceutical composition according to Claim 6, wherein the hyperesthesia is allodynia, and the sensory disorder is dysesthesia or paresthesia.

8. The pharmaceutical composition according to Claim 1 or 2, wherein the peripheral neuropathy is autonomic neuropathy attributable to peripheral neuropathy.

9. The pharmaceutical composition according to Claim 8, wherein a symptom caused by the autonomic neuropathy is urinary disturbance, sweating disorder, orthostatic hypotension, constipation, or paralytic ileus.

10. The pharmaceutical composition according to any one of Claims 1 to 9, wherein the peripheral neuropathy is peripheral neuropathy having symptoms that manifest in the four limbs.

11. The pharmaceutical composition according to any one of Claims 1 to 10,
wherein the alkalinizing agent is a pharmaceutically acceptable salt of citric acid or a hydrate thereof, or a mixture thereof.

12. The pharmaceutical composition according to any one of Claims 1 to 11,
wherein the alkalinizing agent is sodium citrate, potassium citrate, a hydrate of sodium citrate or potassium citrate, or a mixture thereof.

13. The pharmaceutical composition according to any one of Claims 1 to 12,
wherein the alkalinizing agent comprises a mixture of sodium citrate or a hydrate thereof, and potassium citrate or a hydrate thereof.

14. The pharmaceutical composition according to any one of Claims 1 to 13,
wherein the alkalinizing agent is sodium citrate or a hydrate thereof.

15. The pharmaceutical composition according to any one of Claims 1 to 14, wherein the pharmaceutical composition is a tablet.

16. The pharmaceutical composition according to any one of Claims 1 to 10, wherein the alkalinizing agent is sodium bicarbonate.

17. The pharmaceutical composition according to any one of Claims 1 to 16, wherein the peripheral neuropathy is peripheral neuropathy induced by diabetes.

FIG. 1

Day21

N=7-8 mean ± S.E.M. ****; p<0.0001, * ; p<0.05. Unpaired-t test.

## INTERNATIONAL SEARCH REPORT

| International application No. |
| --- |
| **PCT/JP2024/006205** |

**A. CLASSIFICATION OF SUBJECT MATTER**

*A61K 45/00*(2006.01)i; *A61K 9/20*(2006.01)i; *A61K 31/194*(2006.01)i; *A61K 33/10*(2006.01)i; *A61P 13/02*(2006.01)i; *A61P 25/02*(2006.01)i; *A61P 29/00*(2006.01)i

FI: A61K45/00; A61K33/10; A61K31/194; A61P25/02; A61P29/00; A61P13/02; A61K9/20

According to International Patent Classification (IPC) or to both national classification and IPC

**B. FIELDS SEARCHED**

Minimum documentation searched (classification system followed by classification symbols)

A61K45/00; A61K9/20; A61K31/194; A61K33/10; A61P13/02; A61P25/02; A61P29/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| A | GUINDON, J. et al. Optimization of a cisplatin model of chemotherapy-induced peripheral neuropathy in mice: use of vitamin C and sodium bicarbonate pretreatments to reduce nephrotoxicity and improve animal health status. Molecular Pain. 2014 (filed date), 10, 56, pp. 1-14<br>in particular, abstract, fig. 1, 2 | 1-17 |
| A | 牛尾聡一郎,他2名.「漢方薬と抗がん剤誘発神経障害」オキサリプラチンによる末梢神経障害に対する牛車腎気丸の効果に関する基礎的エビデンス.日薬理誌.2014, vol. 143, pp. 126-130, (USHIO, Soichiro et al. Basic evidence for efficacy of Goshajinkigan on oxaliplatin-induced neuropathy. Folia Pharmacologica Japonica.)<br>in particular, abstract, fig. 1, 2 | 1-17 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **18 April 2024** | **07 May 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| | International application No. |
|---|---|
| | **PCT/JP2024/006205** |

**C.    DOCUMENTS CONSIDERED TO BE RELEVANT**

| Category* | Citation of document, with indication, where appropriate, of the relevant passages | Relevant to claim No. |
|---|---|---|
| A | 山田果琳,他3名. 抗がん薬パクリタキセル誘発末梢神経障害性疼痛モデルマウスに対するミロガバリンの作用検討. 第40回鎮痛薬・オピオイドペプチドシンポジウム プログラム・抄録集. 2021, p. 44, non-official translation (YAMADA, Karin et al. Study of Action of Mirogabalin in Anti-Cancer Drug Paclitaxel-Induced Peripheral Neuropathy Pain Model Mice. Program and Abstracts of the 40th Annual Meeting of Japanese Narcotics Research Conference.) entire text | 1-17 |
| A | WO 2020/050253 A1 (NIPPON CHEMIPHAR CO., LTD.) 12 March 2020 (2020-03-12) in particular, claims | 1-17 |
| A | WO 2018/066532 A1 (HOSHI UNIVERSITY) 12 April 2018 (2018-04-12) in particular, claims, examples | 1-17 |

Form PCT/ISA/210 (second sheet) (July 2022)

**INTERNATIONAL SEARCH REPORT**
Information on patent family members

International application No.

**PCT/JP2024/006205**

| Patent document cited in search report | Publication date (day/month/year) | Patent family member(s) | Publication date (day/month/year) |
|---|---|---|---|
| WO 2020/050253 A1 | 12 March 2020 | US 2021/0205326 A1<br>EP 3848051 A1 | |
| WO 2018/066532 A1 | 12 April 2018 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023027474 A
- WO 2018193648 A
- WO 2018193752 A
- WO 2020080451 A

**Non-patent literature cited in the description**

- **CHAPLAN SR** ; **BACH FW** ; **POGREL JW** ; **CHUNG JM** ; **YAKSH TL**. Quantitative assessment of tactile allodynia in the rat paw.. *J Neurosci Methods.*, July 1994, vol. 53 (1), 55, 63